# EUROPEAN PATENT APPLICATION

(11) **EP 1 900 737 A1**
(43) Date of publication of application: **19.03.2008**
(21) Application number: 06119883.4
(22) Date of filing: 31.08.2006
(51) Int. Cl.: C07D 453/02, A61K 31/439, A61P 11/06

(54) **Polymorph of (R)-3-(2-hydroxy-2,2-diphenyl-acetoxy)-1-(isoxazol-3-ylcarbamoyl-methyl)-1-azonia-bicyclo-[2.2.2]octane bromide**

(71) Applicant: Novartis AG, 4002 Basel (CH)
(72) Inventor: The designation of the inventor has not yet been filed
(74) Representative: McLean, Craig Sutherland

(57) **Abstract**

A novel polymorphic crystal form of (R)-3-(2-hydroxy-2,2-diphenyl-acetoxy)-1-(isoxazol-3-ylcarbamoyl-methyl)-1-azonia-bicyclo-[2.2.2]octane bromide, designated crystal form Talpha. Methods for preparing same and the use of the crystal form as the active ingredient of medicaments for the treatment of inflammatory or obstructive airways diseases are also described.

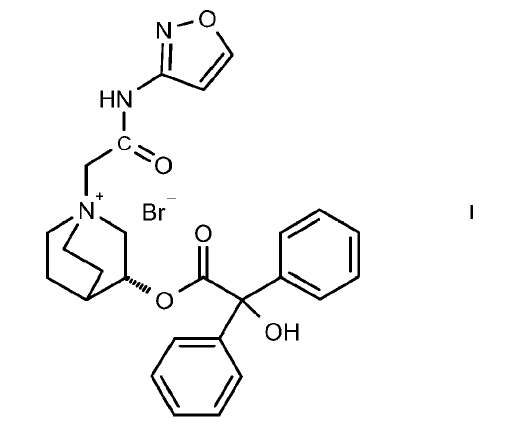

## Description

This invention relates to a new polymorphic crystal form of (R)-3-(2-hydroxy-2,2-diphenyl-acetoxy)-1-(isoxazol-3-ylcarbamoyl-methyl)-1-azonia-bicyclo-[2.2.2]octane bromide, designated crystal form Talpha, and methods for preparing same.

The compound (R)-3-(2-hydroxy-2,2-diphenyl-acetoxy)-1-(isoxazol-3-ylcarbamoyl-methyl)-1-azonia-bicyclo-[2.2.2]octane bromide has the chemical structure of formula I

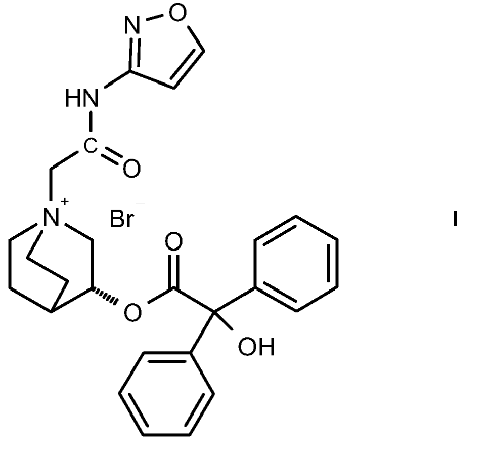

(R)-3-(2-Hydroxy-2,2-diphenyl-acetoxy)-1-(isoxazol-3-ylcarbamoyl-methyl)-1-azonia-bicyclo-[2.2.2]octane bromide, herein the "compound of formula I" is a potent muscarinic antagonist, more specifically a muscarinic M3 receptor antagonist. It inhibits acetylcholine-induced contraction of smooth muscle in e.g. respiratory tract, digestive tract and urinary systems.

The compound of formula I is prepared by the process described in international patent application WO 2004/96800, the contents of which is incorporated herein by reference.

This compound has been investigated for use as a pharmaceutical. The existence of various crystallisation polymorphic forms of the compound has been explored in order to determine the most appropriate form of the compound for the proposed use.

A novel crystal form of the compound of formula I has now been isolated and designated crystal form Talpha. This crystal form has very good stability, facilitating its use in the preparation of pharmaceutical dosage forms.

Accordingly, the present invention provides in one aspect crystalline (R)-3-(2-hydroxy-2,2-diphenyl-acetoxy)-1-(isoxazol-3-ylcarbamoyl-methyl)-1-azonia-bicyclo-[2.2.2]octane bromide designated crystal form Talpha. This crystal form is substantially anhydrous.

In a second aspect the invention provides crystalline (R)-3-(2-hydroxy-2,2-diphenyl-acetoxy)-1-(isoxazol-3-ylcarbamoyl-methyl)-1-azonia-bicyclo-[2.2.2]octane bromide designated crystal form Talpha characterised by a melting point, by Differential Scanning Calorimetry, of about 208°C with simultaneous decomposition.

In a third aspect the invention provides crystalline (R)-3-(2-Hydroxy-2,2-diphenyl-acetoxy)-1-(isoxazol-3-ylcarbamoyl-methyl)-1-azonia-bicyclo-[2.2.2]octane bromide in crystal form Talpha having the following characteristic diffraction lines (2θ in angular degrees ± 0.2°) in the X-ray diffraction pattern thereof: 5.7 °, 15.7 °, 18.5 °, 19.8 °, 21.1 °, 22.2 ° and 24.0°.

In a fourth aspect the invention provides a pharmaceutical composition comprising, as active ingredient, an effective amount of crystalline (R)-3-(2-hydroxy-2,2-diphenyl-acetoxy)-1-(isoxazol-3-ylcarbamoyl-methyl)-1-azonia-bicyclo-[2.2.2]octane bromide, optionally together with a pharmaceutically acceptable carrier. Preferably the composition is in inhalable form.

In a fifth aspect the invention concerns the use of crystalline (R)-3-(2-hydroxy-2,2-diphenyl-acetoxy)-1-(isoxazol-3-ylcarbamoyl-methyl)-1-azonia-bicyclo-[2.2.2]octane bromide for the preparation of a medicament for the treatment of an inflammatory or obstructive airways disease.

In a sixth aspect the invention provides a method of preparing crystalline (R)-3-(2-hydroxy-2,2-diphenyl-acetoxy)-1-(isoxazol-3-ylcarbamoyl-methyl)-1-azonia-bicyclo-[2.2.2]octane bromide which comprises crystallising (R)-3-(2-hydroxy-2,2-diphenyl-acetoxy)-1-(isoxazol-3-ylcarbamoyl-methyl)-1-azonia-bicyclo-[2.2.2]octane bromide from a solution thereof in acetone.

In a seventh aspect, the invention provides a method of preparing crystalline (R)-3-(2-hydroxy-2,2-diphenyl-acetoxy)-1-(isoxazol-3-ylcarbamoyl-methyl)-1-azonia-bicyclo-[2.2.2]octane bromide which comprises crystallizing (R)-3-(2-hydroxy-2,2-diphenyl-acetoxy)-1-(isoxazol-3-ylcarbamoyl-methyl)-1-azonia-bicyclo-[2.2.2]octane bromide from a solution thereof in a mixture of methanol and ethanol.

Terms used in the specification has the following meanings:
"Polymorphism" as used herein is the ability of a compound to crystallize into more than one distinct crystal species. Polymorphs (or crystalline modifications) have an identical chemical structure but often quite different physicochemical properties. Polymorphs include enantiotropic polymorphs and monotropic polymorphs.
"Amorphous" as used herein describes a non-ordered solid state, which may appear during manufacture of the drug substance (crystallization step, drying, milling) or the drug product (granulation, compression). The X-ray powder diffraction pattern of an amorphous solid exhibits no sharp peaks.
"Pseudopolymorph" is a solvate or hydrate of a compound that, like polymorphs, have different physical properties, however, unlike polymorphs, have a different chemical composition. In other words it is a crystal form that incorporates either stoichiometric of non-stoichiometric amounts of a water (in the case of a hydrate) or another solvent (in the case of a solvate).
"Crystallographically pure" as used herein in relation to a crystal form means the crystal form contains at most about 1 % (w/w) of another form. Thus e.g. "crystallographically pure crystal form Talpha" contains ≤ about 1 %(w/w) of another form.

Throughout this specification and in the claims that follow, unless the context requires otherwise, the word "comprise", or variations such as "comprises" or "comprising", will be understood to imply the inclusion of a stated integer or group of integers but not the exclusion of any other integer or group of integers.

The new polymorphic crystal form of the present invention shall now be described with reference to the accompanying drawings. In the drawings:
Figure 1 is an X-ray diffraction pattern for crystal form Talpha.
Figure 2 is an X-ray diffraction pattern for amorphous (R)-3-(2-hydroxy-2,2-diphenyl-acetoxy)-1-(isoxazol-3-ylcarbamoyl-methyl)-1-azonia-bicyclo-[2.2.2]octane bromide.
Figure 3 is a Raman spectrum for crystal form Talpha.
Figure 4 is a Raman spectrum for amorphous (R)-3-(2-hydroxy-2,2-diphenyl-acetoxy)-1-(isoxazol-3-ylcarbamoyl-methyl)-1-azonia-bicyclo-[2.2.2]octane bromide.
Figure 5 is an IR spectrum for crystal form Talpha.
Figure 6 is an IR spectrum for amorphous (R)-3-(2-hydroxy-2,2-diphenyl-acetoxy)-1-(isoxazol-3-ylcarbamoyl-methyl)-1-azonia-bicyclo-[2.2.2]octane bromide.

The present invention provides crystalline (R)-3-(2-hydroxy-2,2-diphenyl-acetoxy)-1-(isoxazol-3-ylcarbamoyl-methyl)-1-azonia-bicyclo-[2.2.2]octane bromide designated crystal form Talpha.

The compound of formula I in its amorphous form may be prepared in accordance with the method given in Example 21 of international patent application WO 2004/96800. This compound is useful as an active ingredient in medicaments for the treatment of inflammatory or obstructive airways diseases.

Crystal form Talpha is substantially anhydrous. This is important when formulating medicaments that contain (R)-3-(2-hydroxy-2,2-diphenyl-acetoxy)-1-(isoxazol-3-ylcarbamoyl-methyl)-1-azonia-bicyclo-[2.2.2]octane bromide as an active ingredient as the presence of even small amounts of water can cause the active ingredient to convert to the less thermodynamically stable monohydrate form.

While the first recovery of the compound of formula I in crystalline form occurred several years after the first synthesis of the compound, initially obtained only in amorphous form, it has now been found since its first crystallization that the compound can be induced to crystallize from the amorphous form quite readily. The crystalline material has thus now become easily accessible, using a variety of experimental conditions extending beyond the initially used recrystallization conditions, which involved crystallising the compound from acetonitrile.

Crystal form Talpha may be prepared by crystallising the compound of formula I from a solution thereof in acetone, for example by equilibrating the compound in that solvent over 90 minutes at 35° C ± 01, or analogously such as hereinafter described in Example 1. Preferably the solution has a concentration of between 5% and 30 % by weight, preferably between about 15% and 25% by weight, and most preferably about 20% by weight. Water may be added to achieve dissolution of the compound. Preferably, the solution is cooled to 0°C from about 50°C to 70°C, and preferably from about 60°C to 65°C, in about 0.5 to 24 hours, more specifically 2 to 4 hours, and preferably 3 hours.

Alternatively, crystal form Talpha may be prepared by crystallising the compound of formula I from a solution thereof in a mixture of methanol and ethanol. Preferably, the solvent mixture contains methanol and ethanol in equal parts by mass, and crystallization is carried out from a solution of the compound in the mixture with a concentration of between 5 % and 25 % by weight, more preferably between 10 and 20% by weight, and most preferably around 15% by weight. Preferably, the solution is cooled to 0°C from about 60°C to 75°C, and preferably from about 60°C to 65°C, in about 4 to 24 hours, more specifically 8-16 hours, and preferably 12 hours.

For subsequent crystallisations it is preferred to add "seeds" of the crystalline material to the solution in order to induce crystallization such as hereinafter described in Example 1.

The compound of formula I in crystalline form can readily be isolated, it can e.g. be filtered off or centrifuged from the crystallization medium. For the preparation of crystal form Talpha working up may be carried out generally using known procedures for the separation of the crystallisate from the mother liquor, for example by filtration, with or without the assistance of pressure and/or vacuum, or by centrifugation, and subsequent drying of the crystallisate.

Amorphous parts can be converted into the crystalline form by suitable art-known methods.

Crystal form Talpha can be characterised in a variety of ways.

Crystal form Talpha has a melting point, by Differential Scanning Calorimetry, of about 208°C with simultaneous decomposition, for example at a heating rate of 10 K/min.

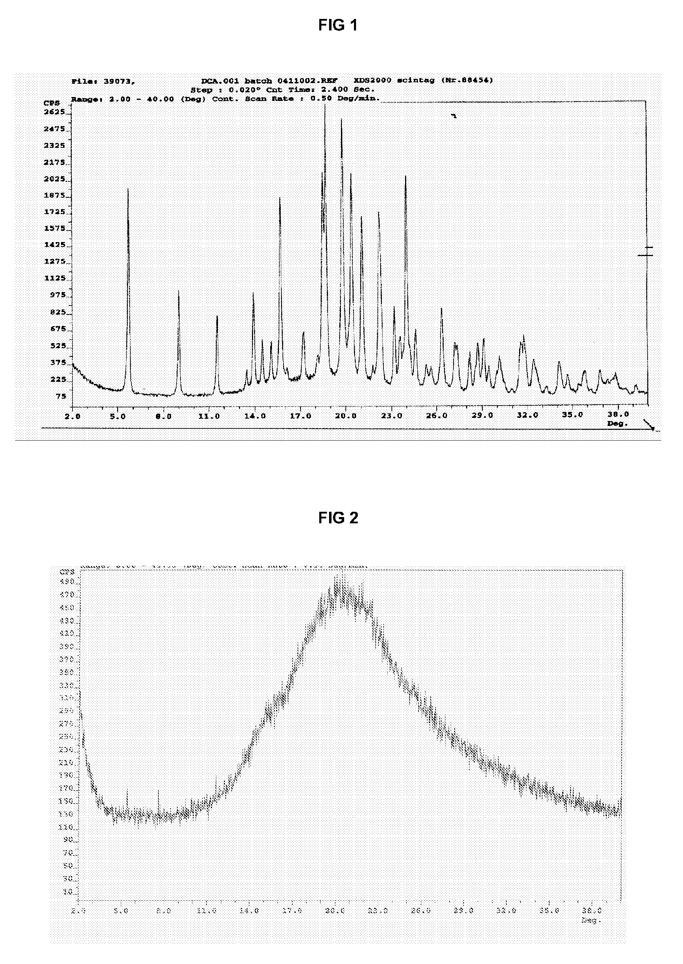

Crystal form Talpha has the characteristic diffraction lines (2θ in angular degrees ± 0.2°) in the X-ray diffraction pattern thereof shown in Figure 1. This is described in more detail in Example 2. The XRPD pattern shows characteristic diffraction lines (2θ in angular degrees ± 0.2°) at 5.7 °, 9.0 °, 11.5 °, 13.9 °, 15.1 °, 15.7 °, 17.2 °, 18.5 °, 18.7 °, 19.8 °, 20.6°, 21.1 °, 22.2°, 23.2 °, 24.0 °, 24.6 °, 26.3 °, 27.2 °, 27.4°, 28.7 °, 29.1 °, 31.6° and 31.8°. The peaks at 5.7 °, 15.7 °, 18.5 °, 19.8 °, 21.1 °, 22.2 ° and 24.0° are the predominant peaks. The peaks at 9.0 °, 11.5 °, 13.9°, 23.2 °, 24.2° and 26.3 ° are also strong although less predominant. The strongest diffraction peak is at 15.7°. The X-ray diffraction pattern for amorphous (R)-3-(2-hydroxy-2,2-diphenyl-acetoxy)-1-(isoxazol-3-ylcarbamoyl-methyl)-1-azonia-bicyclo-[2.2.2]octane bromide does not present any diffraction peaks, as shown in Figure 2, and as such is clearly distinguishable from the XRPD pattern of crystal form Talpha.

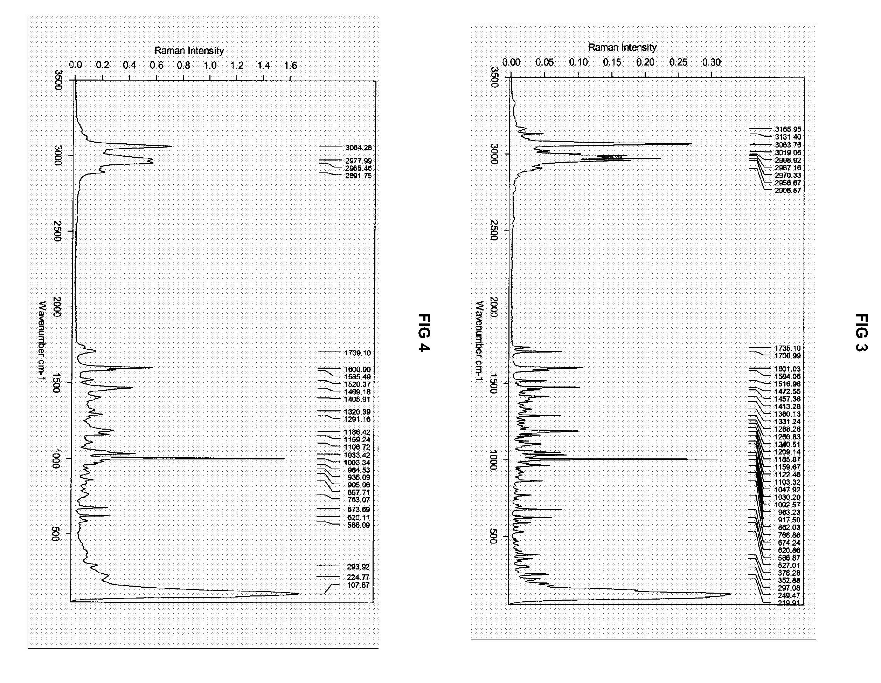

Crystal form Talpha can be characterised by its FT-Raman spectrum. The FT-Raman spectrum of crystal form Talpha is shown in Figure 3. This is described in more detail in Example 3. The FT-Raman spectrum for amorphous (R)-3-(2-hydroxy-2,2-diphenyl-acetoxy)-1-(isoxazol-3-ylcarbamoyl-methyl)-1-azonia-bicyclo-[2.2.2]octane bromide is shown in Figure 4 and is clearly distinguishable from the FT-Raman spectrum of crystal form Talpha.

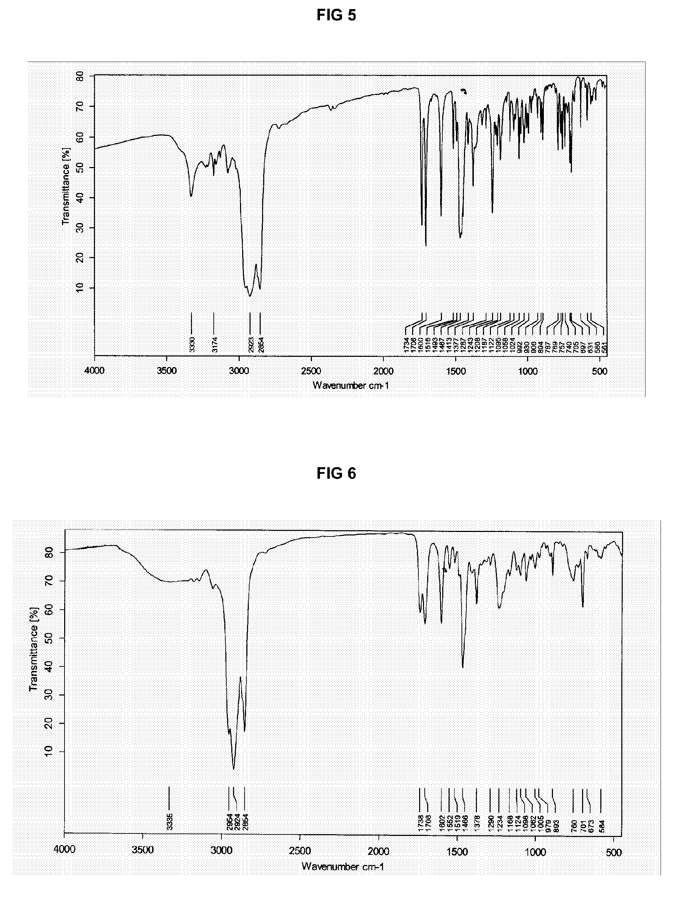

Crystal form Talpha can be characterised by its FT-IR spectrum. The FT-IR spectrum of crystal form Talpha is shown in Figure 5. This is described in more detail in Example 4. The FT-IR spectrum for amorphous (R)-3-(2-hydroxy-2,2-diphenyl-acetoxy)-1-(isoxazol-3-ylcarbamoyl-methyl)-1-azonia-bicyclo-[2.2.2]octane bromide is shown in Figure 6 and is clearly distinguishable from the FT-IR spectrum of crystal form Talpha.

Samples of crystal form Talpha observed by scanning electron microscopy (SEM) reveal a population of large, white, needle-shaped crystals.

A second crystal form of the compound of formula I, designated tbeta, has been obtained by crystallising the compound from a solution thereof in acetone and water (94/6) at 80° C ± 01, but this appears to be a hemihydrate i.e. a pseudopolymorph. A monohydrate, designated tgamma has been obtained by crystallising the compound of formula I at 25° C and at 50° C in the presence of water. A solvate form has also been obtained from dimethylformamide (DMF).

Having regard to its inhibition of acetyl choline binding to M3 muscarinic receptors, the compound of formula I in crystal form Talpha is useful in the treatment of conditions mediated by the muscarinic M3 receptor, particularly those associated with increased parasympathetic tone leading to, for example, excessive glandular secretion or smooth muscle contraction. Treatment in accordance with the invention may be symptomatic or prophylactic.

The affinity (Ki) of the compound of formula I in crystal form Talpha at the human muscarinic acetylcholine M3 receptor can be determined in a competitive filtration binding assay with the radio-labelled antagonist [³H] n-methyl scopolamine methyl chloride (NMS): Membranes prepared from CHO cells stably transfected with human M3 receptor at 10 µg protein/ well are incubated with serial dilutions of the compound of formula I in crystal form Talpha, [³H]NMS (0.25 nM) and assay buffer (20 mM HEPES, 1 mM MgCl₂ at pH 7.4) for 17 hours at room temperature. The assay is carried out in a 250 µL final volume, in the presence of a final dimethyl sulfoxide concentration of 1%. Total binding of [³H]NMS is determined with a corresponding substituted volume of assay buffer. Non-specific binding of [³H] NMS is determined in the presence of 300 nM ipratropium bromide. Following the incubation period, the membranes are harvested onto a Unifilter™ GF/B filter plate containing 0.05 % polyethyleneimine, using a Brandel™ filtration harvester 9600. Filter plates are dried for two hours at 35°C before the addition of Microscint™ 'O' cocktail, and read on a Packard Topcount™ scintillator using a ³H-Scintillation protocol. All IC50s are calculated with the aid of XL-Fit graph package and Kᵢ values derived using the Cheng-Prusoff correction (Cheng Y., Prusoff W. H. (1973) Biochem. Pharmacol. 22 3099-3109).

Having regard to its anti-muscarinic activity, the compound of formula I in crystal form Talpha is useful in the relaxation of bronchial smooth muscle and the relief of bronchoconstriction. Relief of bronchoconstriction can be measured in models such as the in vivo plethysmography models of Chong et al, J. Pharmacol. Toxicol. Methods 1998, 39, 163, Hammelmann et al, Am. J. Respir. Crit. Care Med., 1997, 156, 766 and analogous models. The compound of formula I in crystal form Talpha is therefore useful in the treatment of obstructive or inflammatory airways diseases. In view of its long duration of action, it is possible to administer the compound of formula I in crystal form Talpha once-a-day in the treatment of such diseases. In another aspect, the compound of formula I in crystal form Talpha commonly exhibits characteristics indicating a low incidence of side effects commonly encountered with β₂ agonists such as tachycardia, tremor and restlessness, such compound accordingly being suitable for use in on demand (rescue) treatment as well as prophylactic treatment of obstructive or inflammatory airways diseases.

Inflammatory or obstructive airways diseases to which the present invention is applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant from any previously administered symptomatic asthma therapy.

Other inflammatory or obstructive airways diseases and conditions to which the present invention is applicable include acute lung injury (ALI), adult/acute respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Having regard to its anti-inflammatory activity, in particular in relation to inhibition of eosinophil activation, the compound of formula I in crystal form Talpha is also useful in the treatment of eosinophil related disorders, e.g. eosinophilia, in particular eosinophil related disorders of the airways (e.g. involving morbid eosinophilic infiltration of pulmonary tissues) including hypereosinophilia as it effects the airways and/or lungs as well as, for example, eosinophil-related disorders of the airways consequential or concomitant to Löffler's syndrome, eosinophilic pneumonia, parasitic (in particular metazoan) infestation (including tropical eosinophilia), bronchopulmonary aspergillosis, polyarteritis nodosa (including Churg-Strauss syndrome), eosinophilic granuloma and eosinophil-related disorders affecting the airways occasioned by drug-reaction.

The compound of formula I in crystal form Talpha is also useful in the treatment of inflammatory conditions of the skin, for example psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphisus, epidermolysis bullosa acquisita, and other inflammatory conditions of the skin.

The compound of formula I in crystal form Talpha may also be used for the treatment of other diseases or conditions, in particular diseases or conditions having an inflammatory component, for example, treatment of diseases and conditions of the eye such as conjunctivitis, keratoconjunctivitis sicca, and vernal conjunctivitis, diseases affecting the nose including allergic rhinitis, diseases of the joints such as rheumatoid arthritis and inflammatory bowel disease such as ulcerative colitis and Crohn's disease.

Further, the compound of formula I in crystal form Talpha may also be used for the treatment of cystic fibrosis, pulmonary hypertension and pulmonary fibrosis.

The compound of formula I in crystal form Talpha is also useful as a co-therapeutic agent for use in conjunction with other drug substances for treatment of airways diseases, particularly anti-inflammatory, bronchodilatory, antihistaminic/anti-allergic or anti-tussive drug substances, particularly in the treatment of obstructive or inflammatory airways diseases such as those mentioned hereinbefore, for example as potentiators of therapeutic activity of such drugs or as a means of reducing required dosaging or potential side effects of such drugs. The compound of formula I in crystal form Talpha may be mixed with the other drug in a fixed pharmaceutical composition or it may be administered separately, before, simultaneously with or after the other drug.

Such anti-inflammatory drugs include steroids, in particular glucocorticosteroids such as budesonide, beclamethasone dipropionate, fluticasone propionate, ciclesonide, dexamethasone, flunisolide, mometasone furoate and triamcinolone but also compounds described in WO 02/00679, WO 02/88167, WO 02/12266 and WO 02/100879 (including salts or derivatives thereof such as sodium salts, sulphobenzoates, phosphates, isonicotinates, acetates, propionates, dihydrogen phosphates, palmitates, pivalates or furoates, and, where possible, hydrates); dopamine agonists such as bromocriptine, cabergolin, alpha- dihydroergocryptine, lisuride, pergolide, pramipexol, roxindol, ropinirol, talipexol, tergurid and viozan (including pharmaceutically acceptable salts thereof such as salts of hydrochloric acid, hydrobromic acid, sulphuric acid, phosphoric acid, methanesulphonic acid, acetic acid, fumaric acid, succinic acid, lactic acid, citric acid, tartaric acid and maleic acid); LTB4 antagonists such as those described in US 5451700; LTB4 antagonists such as those described in US 5451700; LTD4 antagonists such as montelukast and zafirlukast; dopamine receptor agonists such as cabergoline, bromocriptine, ropinirole and 4-hydroxy-7-[2-[[2-[[3-(2-phenylethoxy)-propyl]sulfonyl]ethyl]-amino]ethyl]-2(3H)-benzothiazolone and pharmaceutically acceptable salts thereof (the hydrochloride being Viozan^{®} - AstraZeneca); PDE4 inhibitors such as Ariflo^{®} (GlaxoSmith Kline), Roflumilast (Byk Gulden), enprofylline,V-11294A (Napp), BAY19-8004 (Bayer), D-4396 (Sch-351591), (Schering-Plough), Arofylline (Almirall Prodesfarma), PD189659 (Parke-Davis), AWD-12-281 (Asta Medica), BY343, CDC-801 (Celgene), CP-325366, and KW-4490 (Kyowa Hakko Kogyo) (including physiologically acceptable acid addition salts thereof such as salts of hydrochloric acid, hydrobromic acid, sulphuric acid, phosphoric acid, methanesulphonic acid, acetic acid, fumaric acid, succinic acid, lactic acid, citric acid, tartaric acid and maleic acid); A2a agonists such as those described in EP 409595A2, EP 1052264, EP 1241176, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/039762, WO 04/039766, WO 04/045618 and WO 04/046083; and A2b antagonists such as those described in WO 02/42298.

Such bronchodilatory drugs include beta-2 adrenoceptor agonists. Suitable beta-2 adrenoceptor agonists include albuterol (salbutamol), metaproterenol, terbutaline, salmeterol fenoterol, procaterol, and especially, formoterol, carmoterol and pharmaceutically acceptable salts thereof, and compounds (in free or salt or solvate form) of formula I of WO 00/75114, which document is incorporated herein by reference, preferably compounds of the Examples thereof, especially a compound of formula 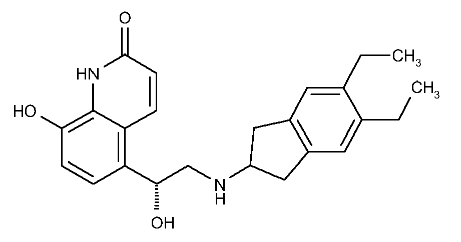 and pharmaceutically acceptable salts thereof, as well as compounds (in free or salt or solvate form) of formula I of WO 04/16601, and also compounds of EP 1440966, JP 05025045, WO 93/18007, WO 99/64035, US 2002/0055651, US 2005/0133417, US 2005/5159448, WO 01/42193, WO 01/83462, WO 02/66422, WO 02/ 70490, WO 02/76933, WO 03/24439, WO 03/42160, WO 03/42164, WO 03/72539, WO 03/91204, WO 03/99764, WO 04/16578, WO 04/22547, WO 04/32921, WO 04/33412, WO 04/37768, WO 04/37773, WO 04/37807, WO 04/39762, WO 04/39766, WO 04/45618 WO 04/46083 , WO 04/80964, EP1460064, WO 04/087142, WO 04/089892, EP 01477167, US 2004/0242622, US 2004/0229904, WO 04/108675, WO 04/108676, WO 05/033121, WO 05/040103, WO 05/044787, WO 05/058867, WO 05/065650, WO 05/066140 and WO 05/07908.

Such bronchodilatory drugs also include other anticholinergic or antimuscarinic agents, such as those described in EP 424021, US 5171744 (Pfizer) and WO 01/04118 (Almirall Prodesfarma) but in particular ipratropium bromide, oxitropium bromide and tiotropium bromide, and beta-2 adrenoceptor agonists such as salbutamol, terbutaline, salmeterol and, especially, formoterol and pharmaceutically acceptable salts thereof.

Suitable dual anti-inflammatory and bronchodilatory drugs include dual beta-2 adrenoceptor agonist / muscarinic antagonists such as those disclosed in US 2004/0167167, US 2004/0242622, US 2005/182092, WO 04/74246 and WO 04/74812.

Suitable antihistaminic/anti-allergic drug substances include acetaminophen, activastine, astemizole, azelastin, bamipin, cetirizine hydrochloride, cexchloropheniramine, chloro-phenoxamine, clemastine fumarate, desloratidine, dimenhydrinate, dimetinden, diphenhydramine, doxylamine, ebastine, emedastin, epinastine, fexofenadine hydrochloride, ketotifen, levocabastin, loratidine, meclizine, mizolastine, pheniramine, promethazine and tefenadine, as well as those disclosed in JP 2004107299, WO 03/099807 and WO 04/026841 (including any pharmacologically acceptable acid addition salts thereof which may exist).

Combinations of the compound of formula I in crystal form Talpha and beta-2 adrenoceptor agonists, steroids, PDE4 inhibitors or LTD4 antagonists are particularly suitable for use in the treatment of asthma.

Combinations of the compound of formula I in crystal form Talpha and beta-2 adrenoceptor agonists, PDE4 inhibitors, LTB4 antagonists are particularly suitable for use in the treatment of COPD.

In accordance with the foregoing, the invention also provides a method for the treatment of an inflammatory condition, particularly an inflammatory or obstructive airways disease, which comprises administering to a subject, particularly a human subject, in need thereof an effective amount of the compound of formula I in crystal form Talpha as hereinbefore described. In another aspect the invention provides the use of the compound of formula I in crystal form Talpha for the manufacture of a medicament for the treatment of an inflammatory condition, particularly an inflammatory or obstructive airways disease.

The compound of formula I in crystal form Talpha may be administered by any appropriate route, e.g. orally, for example in the form of a tablet or capsule; parenterally, for example intravenously; by inhalation, for example in the treatment of inflammatory or obstructive airways disease; intranasally, for example in the treatment of allergic rhinitis; topically to the skin, for example in the treatment of atopic dermatitis; or rectally, for example in the treatment of inflammatory bowel disease.

In a further aspect, the invention also provides a pharmaceutical composition comprising as active ingredient the compound of formula I in crystal form Talpha optionally together with a pharmaceutically acceptable diluent or carrier therefor. The composition may contain a co-therapeutic agent such as a bronchodilatory or anti-inflammatory drug as hereinbefore described. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g. patches. Compositions for inhalation may comprise aerosol or other atomizable formulations or dry powder formulations.

When the composition comprises an aerosol formulation, it preferably contains, for example, a hydro-fluoro-alkane (HFA) propellant such as HFA134a or HFA227 or a mixture of these, and may contain one or more co-solvents known in the art such as ethanol (up to 20% by weight), and/or one or more surfactants such as oleic acid or sorbitan trioleate, and/or one or more bulking agents such as lactose. When the composition comprises a dry powder formulation, it preferably contains, for example, the compound of formula I in crystal form Talpha having a particle diameter up to 10 microns, optionally together with a diluent or carrier, such as lactose, of the desired particle size distribution and a compound that helps to protect against product performance deterioration due to moisture e.g. magnesium stearate, typically 0.05-2.0% magnesium stearate. When the composition comprises a nebulised formulation, it preferably contains, for example, the compound of formula I in crystal form Talpha either dissolved, or suspended, in a vehicle containing water, a co-solvent such as ethanol or propylene glycol and a stabiliser, which may be a surfactant.

The invention includes (A) the compound of formula I in crystal form Talpha in inhalable form, e.g. in an aerosol or other atomisable composition or in inhalable particulate, e.g. micronised, form, (B) an inhalable medicament comprising the compound of formula I in crystal form Talpha in inhalable form; (C) a pharmaceutical product comprising the compound of formula I in crystal form Talpha in inhalable form in association with an inhalation device; and (D) an inhalation device containing the compound of formula I in crystal form Talpha in inhalable form.

Dosages of the compound of formula I in crystal form Talpha employed in practising the present invention will of course vary depending, for example, on the particular condition to be treated, the effect desired and the mode of administration. In general, suitable daily dosages for administration by inhalation are of the order of 0.005 to 10 mg, while for oral administration suitable daily doses are of the order of 0.05 to 100 mg.

The invention is illustrated by the following Examples.

### EXAMPLES

### Example 1

### Preparation of crystal form Talpha

24 g (R)-3-(2-Hydroxy-2,2-diphenyl-acetoxy)-1-(isoxazol-3-ylcarbamoyl-methyl)-1-azonia-bicyclo-[2.2.2]octane bromide prepared by the process described in international patent application WO 2004/96800 is dissolved in 120 ml of acetone at a temperature between 50°C and 60°C, and preferably at around 50 to 55 °C. 1 ml of water is added to achieve dissolution of the compound. The solution is cooled to 45°C, and inoculated with 20 mg of the compound in crystalline form. The suspension is then cooled to 0°C in 3 hours, and the suspension kept under stirring for 2 hours at 0°C. The product is subsequently isolated by filtration, washed with twice 15 g of acetone, and dried at 40°C and plant vacuum for 12 hours. The compound of formula I is obtained in the form of white crystals.

### Example 2

### Preparation of crystal form Talpha

236 g (R)-3-(2-Hydroxy-2,2-diphenyl-acetoxy)-1-(isoxazol-3-ylcarbamoyl-methyl)-1-azonia-bicyclo-[2.2.2]octane bromide prepared by the process described in international patent application WO 2004/96800 are dissolved in a mixture comprising 708 g of methanol and 708 g of ethanol at a temperature of about 60°C. The solution is filtered at a temperature at or slightly above 60°C, and then cooled to 55°C in about 1-2 hours. At 55°C, the solution is inoculated with 200 mg of the compound in crystalline form. The suspension is then kept under stirring at 55°C for one hour, and then cooled to 0°C in 12 hours. The suspension is then kept under stirring for another 6 hours at 0°C. The product is subsequently isolated by filtration, washed with six times 150 g of pre-cooled ethanol at 0°C, and dried at 60°C and full plant vacuum for 8 hours. The compound of formula I is obtained in the form of white crystals of high aspect ratio and needle-like shape.

### Example 3

### Characterisation of crystal form Talpha by X-ray powder diffraction

The X-ray diffraction pattern of crystal form Talpha prepared in accordance with Example 1 is measured using a SCINTAG™ X-ray diffractometer with a CuK alpha radiation source. The X-ray diffraction pattern thus determined is shown in Figure 1 and represented in Table 1 below by the reflection lines and intensities of the most important lines.

**TABLE 1**

| **X-ray diffraction lines and intensities for crystal form Talpha** | | |
|---|---|---|
| **2θ(°)** | **d-spacings (Å)** | **Relative intensity** |
| 5.7 | 15.41 | S |
| 9.0 | 9.81 | M |
| 11.5 | 7.68 | M |
| 13.9 | 6.35 | M |
| 14.5 | 6.10 | L |
| 15.1 | 5.87 | L |
| 15.7 | 5.63 | S |
| 17.2 | 5.15 | L |
| 18.5 | 4.79 | S |
| 18.7 | 4.74 | S |
| 19.8 | 4.48 | S |
| 20.4 | 4.35 | S |
| 21.1 | 4.21 | S |
| 22.2 | 4.00 | S |
| 23.2 | 3.83 | M |
| 24.0 | 3.70 | S |
| 24.6 | 3.62 | L |
| 26.3 | 3.38 | M |
| 27.2 | 3.28 | L |
| 27.4 | 3.26 | L |
| 28.7 | 3.11 | L |
| 29.1 | 3.06 | L |
| 31.6 | 2.83 | L |
| 31.8 | 2.81 | L |

The XRPD pattern shows a strong diffraction peak at 5.7°.

The X-ray diffraction pattern for amorphous (R)-3-(2-hydroxy-2,2-diphenyl-acetoxy)-1-(isoxazol-3-ylcarbamoyl-methyl)-1-azonia-bicyclo-[2.2.2]octane bromide obtained using the same diffractometer with alpha radiation source is shown in Figure 2.

### Example 4

### Characterisation of crystal form Talpha by Raman spectroscopy

The FT-Raman spectrum of crystal form Talpha prepared in accordance with Example 1 is measured using a BRUKER OPTICS RFS 100^{™} spectrometer. The fT-Raman spectrum thus determined is shown in Figure 3 and represented by the reflection lines and intensities of the most important lines: 3131, 3064, 2987, 2970, 2957, 1735, 1707, 1601, 1517, 1473, 1413, 1288, 1185, 1047, 1030, 1003, 963, 862, 674, 621, 378, 353 and 250 cm⁻¹. The FT-Raman spectrum for amorphous (R)-3-(2-Hydroxy-2,2-diphenyl-acetoxy)-1-(isoxazol-3-ylcarbamoyl-methyl)-1-azonia-bicyclo-[2.2.2]octane bromide obtained using the same spectrometer is shown in Figure 4 and represented by the reflection lines and intensities of the most important lines: 3064, 2978, 2955, 1709, 1601, 1469, 1186, 1159, 1033, 1003, 674, 620 and 224 cm⁻¹.

### Example 5

### Characterisation of crystal form Talpha by IR spectroscopy

The FT-IR spectrum of crystal form Talpha prepared in accordance with Example 1 is measured using the transmission KBr technique and a BRUKER OPTICS IFS-55™ Fourier Transform Infrared (FTIR) spectrometer. The FT-IR spectrum thus determined is shown in Figure 5. Major IR bands are recorded at 3330 (broad), 3174, 2923, 1734, 1706, 1600, 1516, 1493, 1467, 1413, 1377, 1287, 1243, 1208, 1187, 1122, 1095, 1058, 1024, 992, 930, 906, 894, 787, 769, 757, 740, 697, 631, 586 and 561 cm⁻¹. The FR-Raman spectrum for amorphous (R)-3-(2-hydroxy-2,2-diphenyl-acetoxy)-1-(isoxazol-3-ylcarbamoyl-methyl)-1-azonia-bicyclo-[2.2.2]octane bromide obtained using the same spectrometer is shown in Figure 6. Major IR bands are recorded at 3330 (broad), 3175, 2923, 1738, 1708, 1602, 1519, 1466, 1378, 1290, 1234, 893, 760 and 673 cm⁻¹.

### Example 6

### Characterisation of crystal form Talpha by scanning electron microscopy

Samples of crystal form Talpha prepared in accordance with Example 1 observed by scanning electron microscopy (SEM) reveal a population of large needle-shaped crystals.

## Claims

1. Crystalline (R)-3-(2-hydroxy-2,2-diphenyl-acetoxy)-1-(isoxazol-3-ylcarbamoyl-methyl)-1-azonia-bicyclo-[2.2.2]octane bromide designated crystal form Talpha.

2. Crystalline (R)-3-(2-hydroxy-2,2-diphenyl-acetoxy)-1-(isoxazol-3-ylcarbamoyl-methyl)-1-azonia-bicyclo-[2.2.2]octane bromide designated crystal form Talpha **characterised by** a melting point, by Differential Scanning Calorimetry, of about 208°C with simultaneous decomposition.

3. Crystalline (R)-3-(2-hydroxy-2,2-diphenyl-acetoxy)-1-(isoxazol-3-ylcarbamoyl-methyl)-1-azonia-bicyclo-[2.2.2]octane bromide designated in crystal form Talpha having the following characteristic diffraction lines (2θ in angular degrees ± 0.2°) in the X-ray diffraction pattern thereof: 5.7 °, 15.7 °, 18.5 °, 19.8 °, 21.1 °, 22.2 ° and 24.0°.

4. Crystalline (R)-3-(2-hydroxy-2,2-diphenyl-acetoxy)-1-(isoxazol-3-ylcarbamoyl-methyl)-1-azonia-bicyclo-[2.2.2]octane bromide according to claim 3, that also has the following characteristic diffraction lines (2θ in angular degrees ± 0.2°) in the X-ray diffraction pattern thereof: 9.0 °, 11.5 °, 13.9°, 23.2 °, 24.2° and 26.3 °.

5. Crystalline (R)-3-(2-hydroxy-2,2-diphenyl-acetoxy)-1-(isoxazol-3-ylcarbamoyl-methyl)-1-azonia-bicyclo-[2.2.2]octane bromide according to any one of claims 1 to 4 for use as a pharmaceutical.

6. A pharmaceutical composition comprising, as active ingredient, an effective amount of crystalline (R)-3-(2-hydroxy-2,2-diphenyl-acetoxy)-1-(isoxazol-3-ylcarbamoyl-methyl)-1-azonia-bicyclo-[2.2.2]octane bromide, optionally together with a pharmaceutically acceptable carrier.

7. A pharmaceutical composition according to claim 6, further comprising one, two, three or more anti-inflammatory, bronchodilatory, antihistaminic/anti-allergic or anti-tussive drug substances.

8. A pharmaceutical composition according to claim 6 or 7 , which is in inhalable form.

9. The use of crystalline (R)-3-(2-hydroxy-2,2-diphenyl-acetoxy)-1-(isoxazol-3-ylcarbamoyl-methyl)-1-azonia-bicyclo-[2.2.2]octane bromide for the preparation of a medicament for the treatment of an inflammatory or obstructive airways disease.

10. A method of preparing crystalline (R)-3-(2-hydroxy-2,2-diphenyl-acetoxy)-1-(isoxazol-3-ylcarbamoyl-methyl)-1-azonia-bicyclo-[2.2.2]octane bromide which comprises crystallising (R)-3-(2-hydroxy-2,2-diphenyl-acetoxy)-1-(isoxazol-3-ylcarbamoyl-methyl)-1-azonia-bicyclo-[2.2.2]octane bromide from a solution thereof in acetone.

11. A method of preparing crystalline (R)-3-(2-hydroxy-2,2-diphenyl-acetoxy)-1-(isoxazol-3-ylcarbamoyl-methyl)-1-azonia-bicyclo-[2.2.2]octane bromide which comprises crystallising (R)-3-(2-hydroxy-2,2-diphenyl-acetoxy)-1-(isoxazol-3-ylcarbamoyl-methyl)-1-azonia-bicyclo-[2.2.2]octane bromide from a solution thereof in a mixture of methanol and ethanol.

12. Crystalline (R)-3-(2-hydroxy-2,2-diphenyl-acetoxy)-1-(isoxazol-3-ylcarbamoyl-methyl)-1-azonia-bicyclo-[2.2.2]octane bromide designated crystal form Talpha substantially as herein described with reference to any one or more of the Examples.
